# EUROPEAN PATENT SPECIFICATION

(11) **EP 2 600 795 B1**
(45) Date of publication and mention of the grant of the patent: **14.03.2018**
(21) Application number: 11743750.9
(22) Date of filing: 05.08.2011
(51) Int. Cl.: A61F 2/07

(54) **STENT GRAFT HAVING A MARKER AND A REINFORCING AND MARKER RING**
STENTPROTHESE MIT EINEM MARKER UND EINEM VERSTÄRKENDEN MARKERRING
ENDOPROTHÈSE AYANT UN MARQUEUR ET UN ANNEAU MARQUEUR DE RENFORCEMENT

(30) Priority: 05.08.2010 AU 2010210022
(43) Date of publication of application: 12.06.2013
(60) Divisional application: 18155372.8
(73) Proprietor: Cook Medical Technologies LLC, Bloomington, IN 47404 (US)
(72) Inventor: DUCKE, Werner, Dieter, Eight Mile Plains, Queensland 4113 (AU); HARTLEY, David, Ernest, Wannanup, West Australia 6210 (AU); KING, Chantelle, Kelvin Grove, Queensland 4059 (AU)
(74) Representative: Georgiou, Sarah Caroline
(86) International application number: PCT/US2011/046728
(87) International publication number: WO 2012/019090

(56) References cited:
- WO-A1-2010/030370
- WO-A2-97/37616
- WO-A2-2004/075789
- US-A1- 2006 004 440

## Description

### Technical Field

This invention relates to a medical device and more particularly to a reinforcing ring used in a stent graft device.

### Background Art

Stent grafts are used to bridge a defect in the vasculature of a patient and can be deployed into the vasculature endovascularly. This requires that the device can be constrained into a small diameter delivery device and be able to expand, or be expanded, when released within the vasculature.

Where there are side branches to the vasculature it may be necessary to provide an aperture in the stent graft, known as a fenestration, to enable access from a deployed stent graft to that side branch. Such a fenestration may be reinforced with a peripheral circular ring stitched to the graft material around the fenestration.

PCT Publication WO 2005/034808 entitled "Fenestrated Stent Graft" describes the use of resilient reinforcing rings around peripheries of fenestrations in stent grafts.

To obtain a good seal of a branch stent graft within the fenestration an inflatable balloon can be used to expand the branch stent graft into the fenestration and for this purpose the reinforcing ring must be able to resist expansion of its diameter. At the same time the ring must be resilient so that it can be distorted into its constrained deployment configuration but when released expand back to its circular configuration. In this specification the term resilient, when used in relation to a wire used to manufacture a reinforcing ring, refers to a wire which is substantially inextensible but which has a spring function so that when distorted and released returns to substantially its original configuration.

This invention will be discussed in relation to the application of a reinforcing ring to a fenestration within the wall of a stent graft and a reinforcing ring to the end of a stent graft but such a ring may have greater applicability such as a peripheral reinforcement of a scalloped end of a stent graft.

Generally such reinforcing rings are manufactured from a metal known as a superelastic metal such as, but not restricted, to a nickel titanium alloy known as nitinol. To form a ring of a superelastic metal the desired final shape is formed from a wire on a former and then the wire on the former is heated above a temperature which sets the wire in the new shape. Upon cooling the ring holds it formed shape and can be distorted and resiliently returns to the formed shape. As a result of the poor radiopacity of nickel-titanium alloys, however, reinforcing rings made from fine nitinol wires can be difficult to visualize from outside the body using non-invasive imaging techniques, such as x-ray fluoroscopy. Consequently, a clinician may not be able to accurately place and/or manipulate a stent graft with a reinforcing ring to a desired position within a body vessel.

PCT Publication WO 2005/034808 referred to above also discloses the use of gold marker beads to assist with providing the necessary visualisation. However, such beads do not precisely indicate the position of fenestrations or other openings.

PCT Publication WO 2010/030370 describes a reinforcement member for a fenestration in a stent graft. The reinforcement member comprises a composite wire formed of a plurality of wires twisted or braided together.

It is the object of this invention to provide a reinforcing ring, or stent graft and reinforcing ring, to overcome the above problem or to at least provide the practitioner with a useful alternative.

Throughout this discussion the term "stent graft" is intended to mean a device which has a tubular body of biocompatible graft material and at least one stent fastened to the tubular body to define a lumen through the stent graft. The stent graft may be bifurcated and have fenestrations, side arms or the like. Other arrangements of stent grafts are also within the scope of the invention.

### DEFINITION PROXIMAL & DISTAL

Throughout this specification the term distal with respect to a portion of the aorta, a deployment device or a prosthesis such as a stent graft is intended to mean the end of the aorta, deployment device or prosthesis such as a stent graft further away in the direction of blood flow from the heart and the term proximal is intended to mean the portion of the aorta, deployment device or end of the prosthesis nearer to the heart. For other lumens within the human or animal body the terms caudal and cranial respectively should be understood.

### Disclosure of The Invention

The invention is disclosed in claim 1 describing a stent graft comprising:
a wall defining a generally tubular lumen, the wall defining an opening, the opening having a circumferential periphery;
a resilient reinforcing wire curved to follow the periphery of the opening; and
a marker winding wound helically around the reinforcing wire,
whereby the marker winding is viewable on an image display system employing electromagnetic radiation so as to indicate the location of the periphery of the opening.

According to an aspect of the invention there is provided a stent graft comprising:
a wall defining a generally tubular lumen, the wall defining a fenestration for providing fluid communication between the lumen and a branch vessel, the fenestration having a circumferential periphery;
a resilient reinforcing wire curved to follow the periphery of the fenestration thereby defining a reinforcing ring;
   a marker winding wound helically around the reinforcing wire of the reinforcing ring such that the marker winding forms a curved helix around the resilient reinforcing wire, the marker winding being wound from a marker wire, wherein the marker wire is orientated transverse to the reinforcing wire in both a deployed shape for use and a squashed shape for loading into a delivery device,
wherein the marker winding defines a curved passageway, the curved passageway having an internal diameter D, wherein the marker winding is helically wound with a pitch providing at least one winding per length D along the reinforcing wire of the reinforcing ring;
whereby the marker winding is viewable on an image display system employing electromagnetic radiation so as to indicate the location of the periphery of the fenestration.

In one form the marker winding defines a curved passageway around the resilient wire of the reinforcing ring, the curved passageway having an internal diameter D, wherein the marker winding is helically wound with a pitch providing at least one winding per length D along the reinforcing wire of the reinforcing ring.

In one form the marker winding is helically wound with a pitch of greater than 60 degrees and in a further form the marker winding is helically wound with a pitch of greater than 75 degrees.

In one form the resilient reinforcing wire is nitinol.

In one form the marker winding is radiopaque.

In one form the marker winding comprises gold wire.

In one form gold wire has a diameter of less than 0.4 mm.

In one form the reinforcing ring is stitched to the wall.

In one form the wound marker winding defines a curved passageway around the resilient wire, the curved passageway having an internal diameter, and the resilient wire having a diameter, the internal diameter of the curved passageway being at least twice the diameter of the resilient wire.

Described herein is a reinforcing and marker ring for a stent graft, the reinforcing and marker ring comprising:
a plurality of turns of a substantially inextensible resilient wire in a circular shape and terminal ends at each end of the wire, the terminal ends each comprising a loop, each loop attachable to a stent graft having an opening or a fenestration so as to substantially lock a peripheral length of the circular shape; and
a marker winding wound helically around the reinforcing wire, the marker winding being viewable on an image display system employing electromagnetic radiation so as to indicate the location of a periphery of the fenestration, wherein the circular shape of the resilient wire, with the marker winding wound around it, is collapsible under radial pressure to form a squashed circular shape for loading into a delivery device, the squashed circular shape self-expanding back to a substantially circular shape upon release from the delivery device.

### Brief Description of the Drawings

This then generally describes the invention but to assist with understanding reference will now be made to the accompanying drawings which show preferred embodiments of the invention.

In the drawings:
Figure 1 shows a stent graft having reinforcing rings in a side wall.
Figure 2a shows a reinforcing ring for use around the fenestration within the stent graft of Figure 1.
Figure 2b shows an alternative embodiment of the reinforcing ring shown in Figure 2a.
Figure 3a shows an enlarged view of the reinforcing ring of Figure 2a and a portion of the stent graft of Figure 1.
Figure 3b shows an enlarged view of the alternative embodiment of the reinforcing ring shown in Figure 2b and portion of the stent graft of Figure 1.
Figure 4a shows the reinforcing ring of Figure 2a in a squashed condition.
Figure 4b shows the reinforcing ring of Figure 2b in a squashed condition.
Figure 5 shows an enlarged view of the portion of the helically wound marker winding of Figures 2b and 3b.
Figure 6 shows an alternative stent graft having a reinforcing ring at a proximal end.

### Best Modes For Carrying Out The Invention

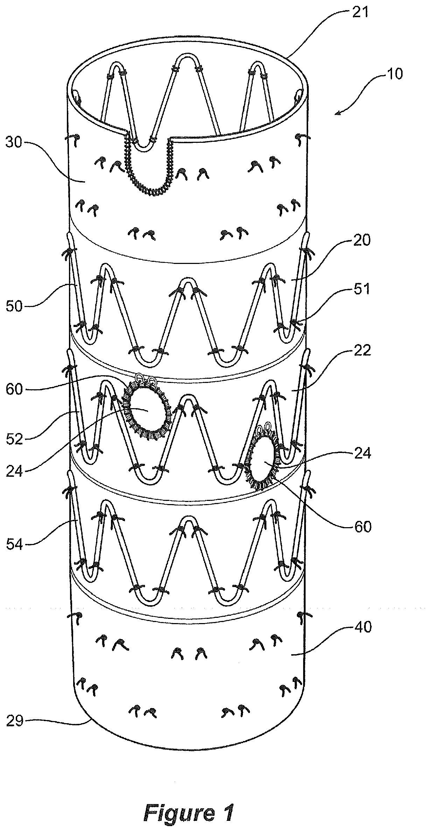

Figures 1, 2a, 3a and 4b show an embodiment of a reinforcing ring according to the present invention.

Referring first to Figure 1, a pair of reinforcing and marker rings 60 around a pair of fenestrations 24 of a stent graft 10, are shown. The stent graft 10 has a tubular body 20 with three stents 50, 52 and 54 stitched onto the tubular body 20 by means of stitches 51. The tubular body has a wall 22 of biocompatible material. Within the wall of the stent graft 10, two reinforcing and marker rings 60 are provided (one, three or any number of reinforcing rings may be provided instead).

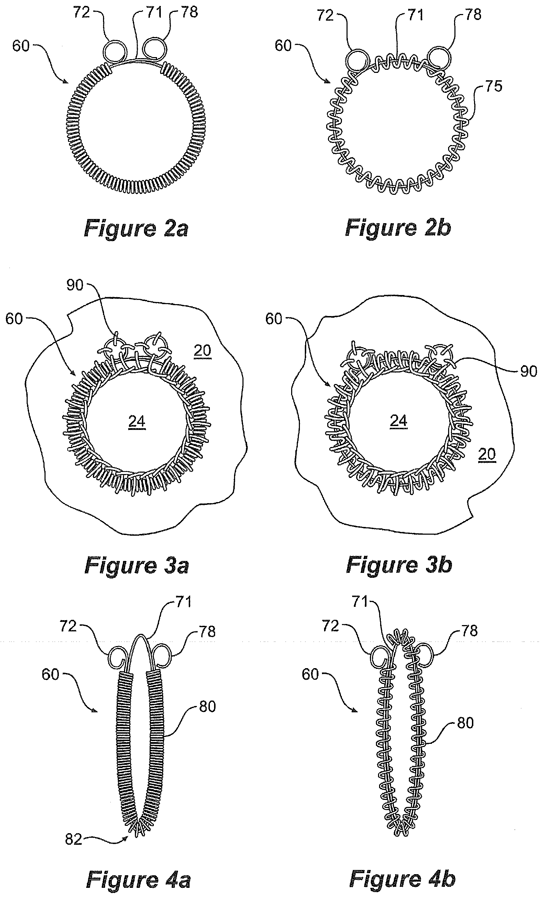

The reinforcing and marker rings 60 are shown separate from the stent graft and in an enlarged view in Figure 2a. Each reinforcing and marker ring 60 comprises a plurality of turns of a substantially inextensible resilient wire 71, such as nitinol wire, in a substantially circular shape. Terminal ends at each end of the wire 71, each comprising a loop 72, 78 are provided. Each loop 72, 78 is attachable to the stent graft 10 so as to substantially lock a peripheral length of the circular shape. This provides the reinforcing and marker ring 60 with a fixed diameter into which, for instance, a self-expanding or balloon expandable stent graft can expanded. A marker winding 80 wound helically around the reinforcing wire is provided. This is shown in Figures 2a and 3a. The marker winding 80 is viewable on an image display system employing electromagnetic radiation so as to indicate the location of a periphery of the fenestration 24.

The circular shape of the resilient wire 71, with the marker winding 80 helically wound around it, is collapsible under radial pressure to form a squashed circular shape, such as is shown in Figure 4a, for loading into a delivery device. The squashed circular shape self-expands back to a substantially circular shape upon release from the delivery device.

It will be particularly noted that in the region 82 as shown in Figure 4a the resilient wire 71 has a sharp bend but in that region the radiopaque marker wire 80 is substantially transverse to the resilient wire and will not, therefore, affect the resiliency of that wire.

The self-expanding property of the reinforcing and marker ring 60 is achieved by the shape memory properties of the wire 71, such as nitinol wire.

Referring now to Figure 3a, it can be seen that the reinforcing and marker ring 60 is attached to the stent graft body 20 by stitching 90.

Figures 2b, 3b and 4b show an alternative embodiment in which the marker winding 80 is wound with a larger pitch.

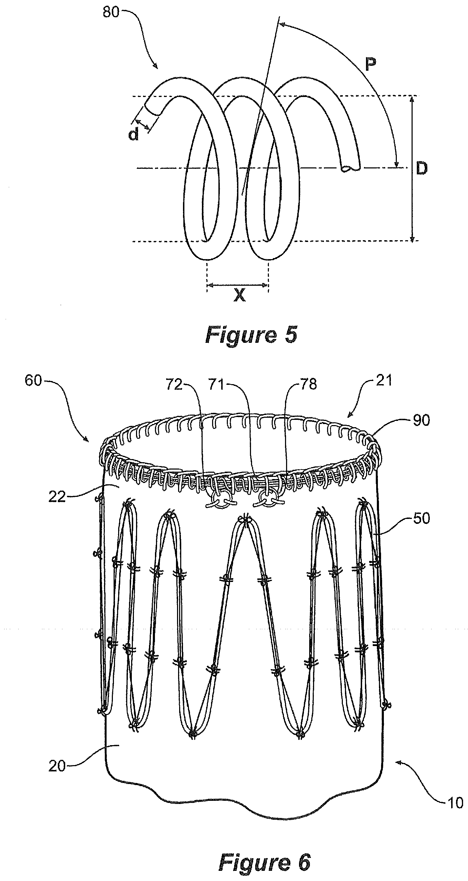

Figure 5 shows an enlarged view of a portion of the helically wound marker winding 80. The marker winding 80 defines a passageway around the resilient wire 71. The passageway has an internal diameter D and is curved to follow the reinforcing wire 71 (not shown in figure 5). The marker winding 80 is helically wound with a pitch providing about two windings per length D along the reinforcing wire in a direction X. The wire 80 can have a diameter d in the range of from 0.2 to 0.5mm.

Various pitches can be used, but pitches providing at least one winding per length D along the reinforcing wire have been found to be particular effective in providing a viewable image on an image display system employing electromagnetic radiation so as to indicate the location of a periphery of the fenestration 24, while at the same time not substantially inhibiting the expansion of the squashed circular shape back to a substantially circular shape upon release from a delivery device.

Pitches of greater than about 60 degrees, and especially greater than 75 degrees, (as is shown in Figure 5 as P) have been found to provide a good viewable image while at the same time not substantially inhibiting the expansion of the squash circular shape back to a substantially circular shaped upon release from a delivery device.

Various materials can be used for the marker winding. For instance, gold has been found to be particularly effective as a radio opaque material suitable for use with x-ray imaging. Gold is also ductile and is readily formed from fine wire into the helical shape required as described above. Various gold wire diameters maybe used, with diameters of less than 0.4mm being particularly effective. In the embodiment illustrated in the drawings, the diameter of the gold wire is approximately 0.2mm and the re-enforcing wire 71 has a diameter of approximately 0.15mm.

In order to assemble a re-enforcing ring such as that illustrated in Figure 3a or 3b, fine gold wire (having a diameter of about 0.2mm) is wound tightly around a 0.5mm wire so as to form a helical winding. The helical winding is then placed onto the loops of nitinol wire 71 to form a complete radiopaque reinforcing and marker ring 60 as is illustrated in Figure 2b. The reinforcing and marker ring 60 is then attached to the wall 22 of the tubular body 20 around the fenestration 24 as is shown in Figure 3b.

Figure 6 shows the construction of an alternative stent graft with a proximal reinforcing ring. The same reference numerals as used in Figure 1 are used for Figure 6 for the corresponding components. The tubular body 20 of the stent graft 10 has a proximal-most external stent 50 stitched onto the tubular body by means of stitches 51. At the proximal end 21 of the stent graft 10 a reinforcing and marker ring 60 is provided. The reinforcing and marker ring 60 comprises two turns of a shape memory wire 71, such as nitinol wire, around the proximal end 21 and loops 72 and 78 at each terminal end of the nitinol wire 71. The loops 72 and 78 prevent the ends of the nitinol wire causing damage to the vasculature in which they are deployed. The two turns of nitinol wire 71 are stitched by means of stitching 90 to the proximal end 21 the tubular body 20.

Marker winding 80, shown in figure 6, is helically wound around the reinforcing wire 71. The marker winding 80, in the form of gold for instance, is viewable on an image display system employing electromagnetic radiation so as to indicate the location of the proximal end 21 of the stent graft 10.

The marker winding 80 described above is provided to assist in visualising position using x-ray fluoroscopy. It should be understood that other non-invasive imaging techniques, such as Magnetic Resonance Imaging (MRI) may also be used and, depending on the type of imaging technique used, different materials can be used for the marker winding 80. While gold is radiopaque and is highly suitable for x-ray fluoroscopy imaging, other material may be used where MRI is to be used.

It will be understood that the term "comprise" and any of its derivatives (e.g. comprises, comprising) as used in this specification is to be taken to be inclusive of features to which it refers, and is not meant to exclude the presence of any additional features unless otherwise stated or implied.

Many modifications and other embodiments of the invention will come to the mind of one skilled in the art having the benefit of the teachings presented in the foregoing descriptions and associated drawings. Therefore, it is understood that the invention is not to be limited to the specific embodiments disclosed, and that modifications and embodiments are intended to be included within the scope of the appended claims.

## Claims

1. A stent graft (10) comprising:
a wall (22) defining a generally tubular lumen, the wall defining a fenestration (24) for providing fluid communication between the lumen and a branch vessel, the fenestration having a circumferential periphery;
a resilient reinforcing wire (71) curved to follow the periphery of the fenestration thereby defining a reinforcing ring (60); and
a marker winding (80) wound helically around the reinforcing wire (71) of the reinforcing ring such that the marker winding forms a curved helix around the resilient reinforcing wire, the marker winding being wound from a marker wire, wherein the marker wire is orientated transverse to the reinforcing wire in both a deployed shape for use and a squashed shape for loading into a delivery device,
wherein the marker winding (80) defines a curved passageway, the curved passageway having an internal diameter D, wherein the marker winding is helically wound with a pitch providing at least one winding per length D along the reinforcing wire of the reinforcing ring;
whereby the marker winding (80) is viewable on an image display system employing electromagnetic radiation so as to indicate the location of the periphery of the fenestration.

2. A stent graft as claimed in claim 1 wherein the marker winding (80) is helically wound with a pitch of greater than 60 degrees.

3. A stent graft as claimed in claim 2 wherein the marker winding (80) is helically wound with a pitch of greater than 75 degrees.

4. A stent graft as claimed in any preceding claim wherein the marker winding comprises a wire having a diameter in the range of from 0.2 to 0.5mm.

5. A stent graft as claimed in claim 1 wherein the resilient reinforcing wire (71) is nitinol.

6. A stent graft as claimed in claim 1 wherein the marker winding (80) is radiopaque.

7. A stent graft as claimed in claim 1 wherein the marker winding (80) comprises gold wire.

8. A stent graft as claimed in claim 1 wherein gold wire has a diameter of less than 0.4 mm.

9. A stent graft as claimed in claim 1 wherein the reinforcing ring (60) is stitched to the wall (22).

10. A stent graft as claimed in claim 1 wherein the helically wound marker winding (80) defines a curved passageway around the resilient wire, the curved passageway having an internal diameter, and the resilient wire having a diameter, the internal diameter of the curved passageway being at least twice the diameter of the resilient wire.

11. A stent graft according to any of claims 1-10:
wherein the resilient reinforcing wire comprises a plurality of turns of a substantially inextensible resilient wire in a circular shape and terminal ends at each end of the wire, the terminal ends each comprising a loop (72, 78), each loop attachable to a stent graft having an opening or a fenestration so as to substantially lock a peripheral length of the circular shape; and wherein the circular shape of the resilient wire (71), with the marker winding (80) wound around it, is collapsible under radial pressure to form a squashed circular shape for loading into a delivery device, the squashed circular shape self-expanding back to a substantially circular shape upon release from the delivery device.

12. A stent graft as claimed in claim 11 wherein the reinforcing ring (60) and marker ring (80) are stitched to the wall of the stent graft (10).

## Patentansprüche

1. Stentprothese (10), umfassend:
eine Wand (22), die ein allgemein röhrenförmiges Lumen definiert, wobei die Wand eine Fensterung (24) zur Bereitstellung einer Fluidverbindung zwischen dem Lumen und einem Zweiggefäß definiert, wobei die Fensterung eine Umfangsperipherie aufweist;
einen nachgiebigen Verstärkungsdraht (71), der so gebogen ist, dass er der Peripherie der Fensterung folgt, wodurch ein Verstärkungsring (60) definiert wird; und
eine Markierungswindung (80), die spiralförmig um den Verstärkungsdraht (71) des Verstärkungsrings gewickelt ist, so dass die Markierungswindung eine gebogene Helix um den nachgiebigen Verstärkungsdraht bildet, wobei die Markierungswindung aus einem Markierungsdraht gewickelt ist, wobei der Markierungsdraht sowohl in einer eingesetzten Gestalt zur Verwendung und in einer gequetschten Gestalt zum Laden in eine Freisetzungsvorrichtung quer zum Verstärkungsdraht orientiert ist,
wobei die Markierungswindung (80) einen gebogenen Durchgang definiert, wobei der gebogene Durchgang einen Innendurchmesser D aufweist, wobei die Markierungswindung spiralförmig mit einer Steigung gewickelt ist, die mindestens eine Windung pro Länge D entlang des Verstärkungsdrahts des Verstärkungsrings bereitstellt;
wobei die Markierungswindung (80) auf einem Bildanzeigesystem, das elektromagnetische Strahlung verwendet, sichtbar ist, um die Position der Peripherie der Fensterung anzuzeigen.

2. Stentprothese nach Anspruch 1, wobei die Markierungswindung (80) spiralförmig mit einer Steigung von mehr als 60 Grad gewickelt ist.

3. Stentprothese nach Anspruch 2, wobei die Markierungswindung (80) spiralförmig mit einer Steigung von mehr als 75 Grad gewickelt ist.

4. Stentprothese nach einem der vorhergehenden Ansprüche, wobei die Markierungswindung einen Draht mit einem Durchmesser im Bereich von 0,2 bis 0,5 mm umfasst.

5. Stentprothese nach Anspruch 1, wobei der nachgiebige Verstärkungsdraht (71) Nitinol ist.

6. Stentprothese nach Anspruch 1, wobei die Markierungswindung (80) röntgenopak ist.

7. Stentprothese nach Anspruch 1, wobei die Markierungswindung (80) Golddraht umfasst.

8. Stentprothese nach Anspruch 1, wobei der Golddraht einen Durchmesser von weniger als 0,4 mm aufweist.

9. Stentprothese nach Anspruch 1, wobei der Verstärkungsring (60) an der Wand (22) festgenäht ist.

10. Stentprothese nach Anspruch 1, wobei die spiralförmig gewickelte Markierungswindung (80) einen gebogenen Durchgang um den nachgiebigen Draht definiert, wobei der gebogene Durchgang einen Innendurchmesser aufweist und der nachgiebige Draht einen Durchmesser aufweist, wobei der Innendurchmesser des gebogenen Durchgangs mindestens das Doppelte des Durchmessers des nachgiebigen Drahts beträgt.

11. Stentprothese nach einem der Ansprüche 1-10:
wobei der nachgiebige Verstärkungsdraht eine Vielzahl von Windungen eines im Wesentlichen nicht ausdehnbaren nachgiebigen Drahts in einer kreisförmigen Gestalt und terminale Enden an jedem Ende des Drahts umfasst, wobei die terminalen Enden jeweils eine Schlaufe (72, 78) umfassen, wobei jede Schlaufe an einer Stentprothese befestigbar ist, die eine Öffnung oder Fensterung aufweist, um eine Umfangslänge der kreisförmigen Gestalt im Wesentlichen zu verriegeln; und wobei die kreisförmige Gestalt des nachgiebigen Drahts (71) mit der um sie gewickelten Markierungswindung (80) unter radialen Druck kollabierbar ist, um eine gequetschte kreisförmige Gestalt zum Laden in eine Abgabevorrichtung zu bilden, wobei die gequetschte kreisförmige Gestalt nach Freigabe von der Abgabevorrichtung zurück in eine im Wesentlichen kreisförmige Gestalt selbstexpandierbar ist.

12. Stentprothese nach Anspruch 11, wobei der Verstärkungsring (60) und der Markerring (80) an der Wand der Stentprothese (10) festgenäht sind.

## Revendications

1. Endoprothèse (10) comprenant :
une paroi (22) délimitant une lumière généralement tubulaire, la paroi délimitant une fenestration (24) permettant une communication fluidique entre la lumière et un vaisseau de ramification, la fenestration ayant une périphérie circonférentielle ;
un fil de renforcement résilient (71) courbé pour suivre la périphérie de la fenestration délimitant de ce fait un anneau de renforcement (60) ; et
un enroulement marqueur (80) enroulé de façon hélicoïdale autour du fil de renforcement (71) de l'anneau de renforcement de sorte que l'enroulement marqueur forme une hélice courbée autour du fil de renforcement résilient, l'enroulement marqueur étant enroulé à partir d'un fil marqueur, le fil marqueur étant orienté transversalement au fil de renforcement à la fois dans une forme déployée lors de l'utilisation et une forme écrasée lors du chargement dans un dispositif de pose,
dans laquelle l'enroulement marqueur (80) délimite un passage courbé, le passage courbé ayant un diamètre interne D, l'enroulement marqueur étant enroulé de façon hélicoïdale avec un pas permettant au moins un enroulement par longueur D le long du fil de renforcement de l'anneau de renforcement ;
moyennant quoi l'enroulement marqueur (80) est visible sur un système d'affichage d'images utilisant un rayonnement électromagnétique de façon à indiquer l'emplacement de la périphérie de la fenestration.

2. Endoprothèse selon la revendication 1, dans laquelle l'enroulement marqueur (80) est enroulé de façon hélicoïdale avec un pas supérieur à 60 degrés.

3. Endoprothèse selon la revendication 2, dans laquelle l'enroulement marqueur (80) est enroulé de façon hélicoïdale avec un pas supérieur à 75 degrés.

4. Endoprothèse selon l'une quelconque des revendications précédentes, dans laquelle l'enroulement marqueur comprend un fil ayant un diamètre dans la plage de 0,2 à 0,5 mm.

5. Endoprothèse selon la revendication 1, dans laquelle le fil de renforcement résilient (71) est du nitinol.

6. Endoprothèse selon la revendication 1, dans laquelle l'enroulement marqueur (80) est radio-opaque.

7. Endoprothèse selon la revendication 1, dans laquelle l'enroulement marqueur (80) comprend un fil en or.

8. Endoprothèse selon la revendication 1, dans laquelle le fil en or a un diamètre inférieur à 0,4 mm.

9. Endoprothèse selon la revendication 1, dans laquelle l'anneau de renforcement (60) est cousu à la paroi (22).

10. Endoprothèse selon la revendication 1, dans laquelle l'enroulement marqueur (80) enroulé de façon hélicoïdale délimite un passage courbé autour du fil résilient, le passage courbé ayant un diamètre interne, et le fil résilient ayant un diamètre, le diamètre interne du passage courbé représentant au moins deux fois le diamètre du fil résilient.

11. Endoprothèse selon l'une quelconque des revendications 1 à 10 :
dans laquelle le fil de renforcement résilient comprend une pluralité de spires d'un fil résilient sensiblement inextensible en une forme circulaire et des extrémités terminales à chaque bout du fil, les extrémités terminales comprenant chacune une boucle (72, 78), chaque boucle pouvant être fixée à une endoprothèse ayant une ouverture ou une fenestration de façon à bloquer sensiblement une longueur périphérique de la forme circulaire ; et dans laquelle la forme circulaire du fil résilient (71), avec l'enroulement marqueur (80) enroulé autour de celle-ci, peut s'affaisser sous l'effet d'une pression radiale afin de former une forme circulaire écrasée pour un chargement dans un dispositif de pose, la forme circulaire écrasée se redéployant en une forme sensiblement circulaire lors de la pose par le dispositif de pose.

12. Endoprothèse selon la revendication 11, dans laquelle l'anneau de renforcement (60) et l'anneau marqueur (80) sont cousus à la paroi de l'endoprothèse (10).
